# EUROPEAN PATENT SPECIFICATION

(11) **EP 2 574 284 B1**
(45) Date of publication and mention of the grant of the patent: **14.08.2019**
(21) Application number: 12186703.0
(22) Date of filing: 28.09.2012
(51) Int. Cl.: A61B 17/04

(54) **Knotless suture anchor**
Knotenloser Nahtanker
Ancrage de sutures sans noeuds

(30) Priority: 30.09.2011 US 201113249941
(43) Date of publication of application: 03.04.2013
(73) Proprietor: DePuy Mitek, Inc., Raynham, MA 02767 (US)
(72) Inventor: Dimatteo, Kristian, Raynham, MA Massachusetts MA 02767 (US); Stephen, Arthur G., Raynham, MA Massachusetts MA 02767 (US); Whittaker, Gregory R., Raynham, MA Massachusetts MA 02767 (US); Capobianco, Mark, Raynham, MA Massachusetts MA 02767 (US)
(74) Representative: Tunstall, Christopher Stephen

(56) References cited:
- EP-A1- 2 335 603
- WO-A1-2009/055075
- US-A1- 2006 235 413
- US-A1- 2007 225 719
- US-A1- 2009 281 581
- US-A1- 2010 049 249

## Description

### Background

This application relates to suture anchors and more particularly to knotless suture anchors.

Suture anchors are commonly employed to attach soft tissue such as tendons or ligaments to bone. For instance, in a rotator cuff repair suture is passed through a detached or damaged portion of a rotator cuff tendon. A suture anchor is implanted into the adjacent bone. By attaching the suture to the anchor the tendon is pulled into contact with the bone to promote adhesion of the tendon to the bone.

Such procedures are often performed arthroscopically through a narrow cannula. This reduces trauma to the patient but makes attachment of the suture to the anchor using a knot more difficult. Knotless suture anchors may be employed which allow a surgeon to tension the suture to a desired degree and then affix to suture to the anchor without having to tie a knot. A typical knotless anchor is shown in US Patent Publication No. 20080033460 wherein the suture is trapped between an inner member and outer member of an anchor in coaxial relation to one another. While such anchors work well their complexity increases manufacturing cost and makes it difficult to form the anchor of bioabsorbable materials which often are more frangible and less strong than metals or traditional polymers.

US 2010/0049249 A1 describes a suture anchor extension which can be attached to an existing eyelet-bearing base suture anchor to create a fully threaded suture anchor. The base suture anchor comprises an eyelet through which suture is passed. The base anchor comprises a body having a point at its distal end and a non-circular driven member at its proximal end. The outer surface of the body is provided with a thread along its length, although the proximal end of the thread ends at a point just distal to the driven member, which enables the driven member to be engaged by the driving member of a driver. The suture anchor extension comprises a tubular body having an outer cylindrical surface, a thread extending along the entire length of the outer surface, and an inner surface surrounding a hollow interior. The inner surface is provided with a non-circular driving member at the distal end of the anchor extension and a non-circular driven member at its proximal end. The driving member is adapted to engage the driven member of the base anchor and driven member is adapted to be engaged by a non-circular driving member formed at the distal end of a cannulated driver. Axially aligned lumens of the extension and the driver, respectively, receive sutures when the base anchor, extension and driver are assembled. The sutures pass through the cannulated driver and are secured to a cleat on the driver handle.

WO 2009/055075 describes an anchor that includes a stopper portion and a fixing portion. The stopper portion includes a piercing point. The piercing point includes a generally hemispherical cavity adapted to receive a suture knot. The piercing point also has a depressed region and a cutting edge. Once a hole has been drilled or pierced into a substrate such as a bone, a cannulated insertion tool can be used that has a suture disposed longitudinally adjacent to a substantially solid shaft. The shaft is used to drive a self-tapping stopper portion into the hole by a leftward rotation of the first shaft. Subsequently, a further portion of the cannulated insertion tool, including a second cannulated shaft coaxially encircling the first substantially solid shaft is used to drive a self-tapping fixing portion into the hole by a rightward rotation of the second shaft until the stopper portion and the fixing portion engage and lock together. Typically, the fixing portion will be installed in a substrate bone so that proximal surface is ultimately disposed substantially flush with an external surface of the bone.

EP 2 335 603 A1 describes a suture anchor having an outer body with an axial bore which receives an inner body for rotation. Suture passes between the inner body and the outer body and rotation of the inner body wraps the suture, locking the suture to it. Rotation of the inner body also effects radial expansion of the outer body to engage to anchor into a bone hole.

### Summary of the Invention

The present invention provides a suture anchor system as defined in appended claim 1, that overcomes the above mentioned and other limitations of the prior art in a simple and elegant design.

Preferred embodiments are defined in the appended dependent claims.

A suture anchor system according to the present invention comprises a distal anchor body having threads thereon to allow for threading of the distal anchor body into bone, and a suture attachment; a driver for driving the distal anchor body into bone, having an elongated driver body with the distal anchor body removably attached to a distal end thereof; and a proximal anchor body having a central bore passing therethrough and one or more purchase enhancements extending from the proximal anchor body. The distal anchor body also has a guide wire trailing proximally from the distal anchor body and releasably attached thereto, wherein the guide wire extends along the driver body. The system also includes a suture passer comprising an elongated flexible member threaded through the suture attachment and having a distal suture capture configuration. The central bore of the proximal anchor body is sized to accommodate the guide wire so that the proximal anchor body can be driven into the bone once the driver has been removed. The one or more purchase enhancements extending from the proximal anchor body are arranged to trap a suture that has been drawn through the suture attachment by the suture passer between the proximal anchor body and the bone.

Preferably, the one or more purchase enhancements comprise screw threads.

### Brief Description of the Drawings

FIG. 1 is a side elevation view of a suture anchor according to the present invention;
FIG. 2 is a side elevation view in cross-section of a tendon, adjacent bone and the anchor of FIG. 1, specifically a distal anchor body thereof in position for entry into a bone hole;
FIG. 3 is a side elevation view in cross-section of a tendon, adjacent bone and the anchor of FIG. 1, specifically the distal anchor body after placement into the bone hole;
FIG. 4 is a side elevation view in cross-section of a tendon, adjacent bone and the anchor of FIG. 1, specifically suture from the tendon being loaded into a suture shuttle threaded through the distal anchor body;
FIG. 5 is a side elevation view in cross-section of a tendon, adjacent bone and the anchor of FIG. 1, specifically the suture from the tendon loaded into the distal anchor body after shuttling via the suture shuttle;
FIG. 6 is a side elevation view in cross-section of a tendon, adjacent bone and the anchor of FIG. 1, specifically the suture being tensioned;
FIG. 7 is a side elevation view in cross-section of a tendon, adjacent bone and the anchor of FIG. 1, specifically a proximal anchor body positioned for entry into the bone hole behind the distal anchor body;
FIG. 8 is a side elevation view in cross-section of a tendon, adjacent bone and the anchor of FIG. 1, specifically the distal anchor being fully inserted into the bone hole;
FIG. 9 is a side elevation view in cross-section of a tendon, adjacent bone and the anchor of FIG. 1, showing the completed deployment of the anchor into the bone;

### Detailed Description

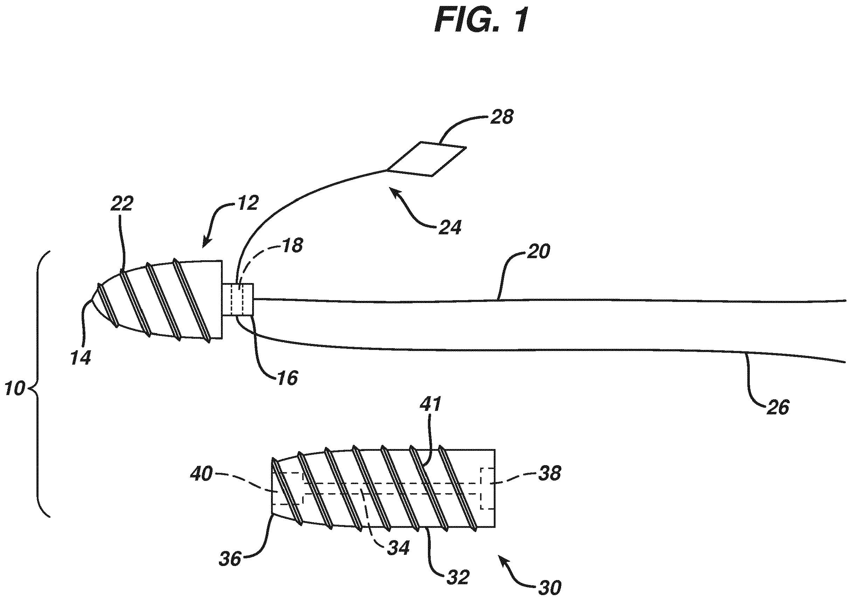

FIG. 1 depicts a knotless suture anchor 10 according to the present invention. It comprises a distal anchor body 12 having a pointed distal tip 14 and a proximally extending extension 16. The extension 16 has a shape, such as hexagonal, for engagement with a driver tool (not shown in FIG. 1) and a suture aperture 18 therethrough for receiving suture (also not shown in FIG. 1). A guide wire 20 extends proximally from the anchor body 12 and is releasably connected thereto, such as by friction or threading. Threads 22 on the body 12 allow for threading of the body 12 into bone. Alternatively, the distal anchor body 12 can be driven awl like into bone in which event the threads 22 can be used for removal or can be omitted altogether. A suture passer 24, such as the CHIA PERCPASSER® available from DePuy Mitek, Inc. of Raynham, MA, is positioned within the suture aperture 18. It comprises an elongated braided NITINOL wire 26 having a kite shaped distal suture capture loop 28.

The suture anchor 10 further comprises a proximal anchor body 30 having external thread 32 and a central axial bore 34 sized to slidably accommodate the guide wire 20. It has a rounded distal end 36 and a proximal driving tool receiving recess 38, preferably hexagonal. It can also be provided with an axial distal socket 40 sized to accommodate the extension 16 of the distal anchor body 12 to allow the proximal anchor body 30 to advance more deeply into a common bone hole as will be described. Preferably, an additional proximal thread start 41is provided, having the same pitch as the thread 32, to provide additional holding in harder cortical bone material such as described is U.S. Patent No. 7,322,978 to West, Jr.

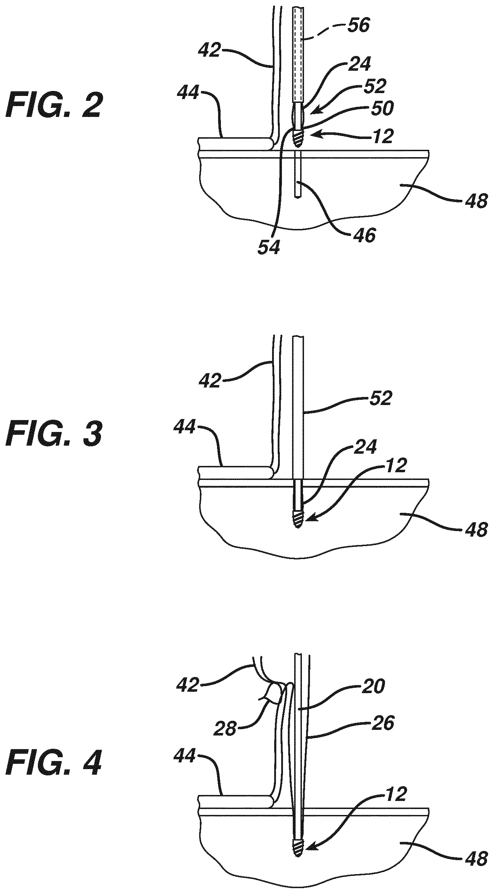

FIGS. 2 to 9 illustrate use of the suture anchor 10 of FIG. 1. A length of suture 42 is passed through a section of tissue 44, such as a tendon in a rotator cuff, and a bone hole 46 is formed in an adjacent bone 48 at a desired location for placement of the anchor 10. The distal anchor body 12, which is disposed on a distal end 50 of a driver 52, is positioned at the bone hole 46 (FIG. 2). The driver 52 has a distal tool end 54 which mates with the proximal extension 16 of the distal anchor body 12 and has one or more axial lumens 56 or grooves for receiving the wire 26. The distal anchor body 12 is then driven into the bone hole 46 (FIG. 3). The driver 52 is removed, leaving the distal anchor 12 in the bone hole 46 with the guide wire 20 extending proximally out of the bone hole 46.

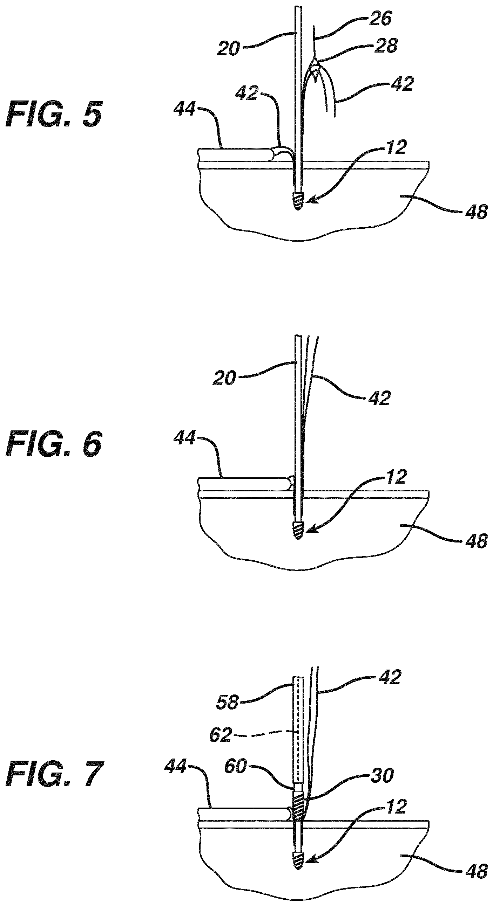

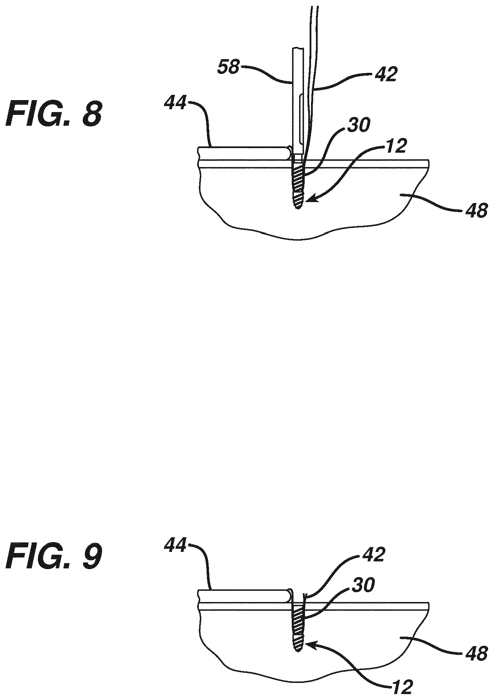

In a preferred arthroscopic procedure, ends of the suture 42, the guide wire 20, the suture capture loop 28 and the other end of the wire 26 all would be extending outside of the patient where the suture 42 would then be loaded into the capture loop 28 (FIG. 4). By drawing on the wire 26 the suture 42 is shuttled through the suture hole 18 of the distal anchor body 12 (FIG. 5). The suture 42 is tensioned to provide the desired position of and tension upon the tissue 44 (FIG. 6). The proximal anchor body 30 is passed distally over the guide wire 20 and positioned at the bone hole 46 (FIG. 7). It is received upon a second driver 58 having a distal tool end 60 which mates with the tool recess 38 of the proximal anchor body 30 and has an axial lumen 62 or groove for receipt of the guide wire 20. Final tension adjustment of the suture 42 can be performed at this time and then the proximal anchor body 30 is driven into the bone hole 46 to lock the suture 42 (FIG. 8). The second driver 58 and the guide wire 20 are removed and the excess suture is 42 trimmed (FIG. 9).

The socket 40 allows the proximal anchor body 30 to be inserted further into the bone hole 30 as its path is not blocked by the extension 16 of the distal anchor body 12. The suture 42 is trapped between the proximal anchor body 30 and the bone hole 46 to lock the suture, and if the proximal anchor body 30 is driven all the way into contract with the distal anchor body 12, with the extension 16 received within the socket 40, the suture 42 can also be trapped between the distal anchor body 12 and proximal anchor body 30 for additional fixation. Optionally, the extension 16 and socket 40 can be configured for engagement to enable additional rotation and further advancement into the bone of the distal anchor body 12.

The FIGS. show a simple suturing technique with a single anchor and a simple loop of suture through tissue in order to focus on the operation of the suture anchors 10. However, the suture anchors 10 of the present invention have utility in more complex configurations such as a dual-row anchoring with a mattress stitch suture configuration, where a row of anchors (often not knotless anchors such as 10) is placed under the tendon and a second row of knotless anchors is placed lateral of the tendon with suture extending up from the first row, through the tendon and to the second row in a crossing pattern whereby to hold the tendon down to the bone.

The novel suture anchors of the present invention may be made from a number of suitable materials including a metallic material, a non-biodegradable polymer, a biodegradable polymer, or a composite of a biodegradable polymer or copolymer and a bioceramic. The term biodegradable as used herein is defined to mean materials that degrade in the body and then are either absorbed into or excreted from the body. The term bioceramic as defined herein is defined to mean ceramic and glass materials that are compatible with body tissue. The bioceramics are preferably biodegradable.

The metallic materials that can be used to manufacture the anchors of the present invention include stainless steel, titanium, alloys of nickel and titanium, or other biocompatible metallic materials.

The non-biodegradable materials that can be used to manufacture the anchors of the present invention include polyethylene, polypropylene, PEEK (polyetheretherketone), or other biocompatible non-absorbable polymers.

The biodegradable polymers that can be used to manufacture the anchors used in the present invention include biodegradable polymers selected from the group consisting of aliphatic polyesters, polyorthoesters, polyanhydrides, polycarbonates, polyurethanes, polyamides and polyalkylene oxides. Preferably, the biodegradable polymers are aliphatic polyester polymers and copolymers, and blends thereof. The aliphatic polyesters are typically synthesized in a ring opening polymerization. Suitable monomers include but are not limited to lactic acid, lactide (including L-, D-, meso and D,L mixtures), glycolic acid, glycolide, .epsilon.-caprolactone, p-dioxanone (1,4-dioxan-2-one), trimethylene carbonate (1,3-dioxan-2-one), .delta.-valerolactone, and combinations thereof.

The bioceramics that can be used in the composite anchors of the present invention include ceramics comprising mono-, di-, tri-, .alpha.-tri-, .beta.- tri-, and tetra-calcium phosphate, hydroxyapatite, calcium sulfates, calcium oxides, calcium carbonates, magnesium calcium phosphates. It is particularly preferred to use a .beta.-tritricalcium phosphate. In addition to bioceramics, bioglasses may also be used in the composite screws. The bioglasses may include phosphate glasses and bioglasses.

Suitable biocompatible synthetic polymers can include polymers selected from the group consisting of aliphatic polyesters, poly(amino acids), copoly(ether-esters), polyalkylene oxalates, polyamides, tyrosine derived polycarbonates, poly(iminocarbonates), polyorthoesters, polyoxaesters, polyamidoesters, polyoxaesters containing amine groups, poly(anhydrides), polyphosphazenes, polyurethanes, poly(ether urethanes), poly(ester urethanes), poly(propylene fumarate), poly(hydroxyalkanoate) and blends thereof.

For the purpose of this invention aliphatic polyesters include, but are not limited to, homopolymers and copolymers of lactide (which includes lactic acid, D-,L- and meso lactide); glycolide (including glycolic acid); .epsilon.- caprolactone; p-dioxanone (1,4-dioxan-2-one); trimethylene carbonate (1,3-dioxan-2-one); alkyl derivatives of trimethylene carbonate; .delta.- valerolactone; .beta.-butyrolactone; .gamma.-butyrolactone; .epsilon.-decalactone; hydroxybutyrate; hydroxyvalerate; 1,4-dioxepan-2-one (including its dimer 1,5,8,12-tetraoxacyclotetradecane-7,14-dione); 1,5-dioxepan-2-one; 6,6-dimethyl-1,4-dioxan-2-one; 2,5-diketomorpholine; pivalolactone; .alpha.,.alpha. diethylpropiolactone; ethylene carbonate; ethylene oxalate; 3-methyl-1,4-dioxane-2,5-dione; 3,3-diethyl-1,4-dioxan-2,5-dione- ; 6,6-dimethyl-dioxepan-2-one; 6,8-dioxabicycloctane-7-one and polymer blends thereof. Additional exemplary polymer or polymer blends include, by non-limiting example, a polydioxanone, a polyhydroxybutyrate-co-hydrox- yvalerate, polyorthocarbonate, a polyaminocarbonate, and a polytrimethylene carbonate. Aliphatic polyesters used in the present invention can be homopolymers or copolymers (random, block, segmented, tapered blocks, graft, triblock, etc.) having a linear, branched or star structure. Poly(iminocarbonates), for the purpose of this invention, are understood to include those polymers as described by Kemnitzer and Kohn, in the Handbook of Biodegradable Polymers, edited by Domb, et. al., Hardwood Academic Press, pp. 251-272 (1997). Copoly(ether-esters), for the purpose of this invention, are understood to include those copolyester-ethers as described in the Journal of Biomaterials Research, Vol. 22, pages 993-1009, 1988 by Cohn and Younes, and in Polymer Preprints (ACS Division of Polymer Chemistry), Vol. 30(1), page 498, 1989 by Cohn (e.g., PEO/PLA). Polyalkylene oxalates, for the purpose of this invention, include those described in U.S. Pat. Nos. 4,208,511; 4,141,087; 4,130,639; 4,140,678; 4,105,034; and 4,205,399. Polyphosphazenes, co-, ter- and higher order mixed monomer based polymers made from L-lactide, D,L-lactide, lactic acid, glycolide, glycolic acid, para-dioxanone, trimethylene carbonate and E-caprolactone such as are described by Allcock in The Encyclopedia of Polymer Science, Vol. 13, pages 31-41, Wiley Intersciences, John Wiley & Sons, 1988 and by Vandorpe, et al in the Handbook of Biodegradable Polymers, edited by Domb, et al., Hardwood Academic Press, pp. 161-182 (1997). Polyanhydrides include those derived from diacids of the form HOOC--C.sub.6H.subA--O--(-CH.sub.2).sub.m--O--C.sub.6H.sub4--COOH, where "m" is an integer in the range of from 2 to 8, and copolymers thereof with aliphatic alpha-omega diacids of up to 12 carbons. Polyoxaesters, polyoxaamides and polyoxaesters containing amines and/or amido groups are described in one or more of the following U.S. Pat. Nos. 5,464,929; 5,595,751; 5,597,579; 5,607,687; 5,618,552; 5,620,698; 5,645,850; 5,648,088; 5,698,213; 5,700,583; and 5,859,150. Polyorthoesters such as those described by Heller in Handbook of Biodegradable Polymers, edited by Domb, et al., Hardwood Academic Press, pp. 99-118 (1997).

The invention has been described with reference to the preferred embodiments. Obviously, modifications and alterations will occur to others upon reading and understanding the preceding detailed description. It is intended that the invention be construed as including all such modifications and alterations insofar as they come within the scope of the appended claims.

## Claims

1. A suture anchor system comprising:
a distal anchor body (12) having threads (22) thereon to allow for threading of the distal anchor body (12) into bone, and a suture attachment (18);
a driver (52) for driving the distal anchor body (12) into bone, having an elongated driver body with the distal anchor body (12) removably attached to a distal end (50) thereof; and
a proximal anchor body (30) having a central bore (34) passing therethrough and one or more purchase enhancements (32) extending from the proximal anchor body (30);
**characterized in that:**
the distal anchor body (12) also has a guide wire (20) trailing proximally from the distal anchor body (12) and releasably attached thereto, wherein the guide wire (20) extends along the driver body;
the system includes a suture passer (24) comprising an elongated flexible member (26) threaded through the suture attachment (18) and having a distal suture capture configuration (28);
the central bore (34) of the proximal anchor body (30) is sized to accommodate the guide wire (20) so that the proximal anchor body (30) can be driven into the bone once the driver (52) has been removed; and
the one or more purchase enhancements (32) extending from the proximal anchor body (30) are arranged to trap a suture (42) that has been drawn through the suture attachment (18) by the suture passer (24) between the proximal anchor body (30) and the bone.

2. A suture anchor system according to claim 1 wherein the suture capture configuration comprises a loop (28) through which suture (42) can be passed.

3. A suture anchor system according to claim 1 wherein the one or more purchase enhancements (32) comprise screw threads (41).

## Patentansprüche

1. Nahtankersystem, umfassend:
einen distalen Ankerkörper (12) mit Gewinden (22) darauf, damit der distale Ankerkörper (12) in den Knochen geschraubt werden kann, und eine Nahtbefestigung (18);
einen Treiber (52) zum Treiben des distalen Ankerkörpers (12) in den Knochen, mit einem länglichen Treiberkörper, wobei der distale Ankerkörper (12) lösbar an einem distalen Ende (50) davon befestigt ist; und
einen proximalen Ankerkörper (30) mit einer mittigen Bohrung (34), die durch ihn hindurch verläuft, und mit einer oder mehreren Halteverstärkungen (32), die sich vom proximalen Ankerkörper (30) erstrecken;
**dadurch gekennzeichnet, dass:**
der distale Ankerkörper (12) auch einen Führungsdraht (20) aufweist, der proximal dem distalen Ankerkörper (12) nachläuft und lösbar daran befestigt ist, wobei sich der Führungsdraht (20) entlang des Treiberkörpers erstreckt;
das System eine Nahtführung (24) aufweist, die ein längliches flexibles Element (26) umfasst, das durch die Nahtbefestigung (18) gefädelt ist und eine distale Nahterfassungskonfiguration (28) aufweist;
die mittige Bohrung (34) des proximalen Ankerkörpers (30) eine Größe zur Aufnahme des Führungsdrahts (20) aufweist, so dass der proximale Ankerkörper (30) in den Knochen getrieben werden kann, nachdem der Treiber (52) entfernt wurde; und
die eine oder die mehreren Halteverstärkungen (32), die sich vom proximalen Ankerkörper (30) erstrecken, zum Einfangen eines Nahtmaterials (42) angeordnet sind, das von der Nahtführung (24) durch die Nahtbefestigung (18) zwischen dem proximalen Ankerkörper (30) und dem Knochen gezogen wurde.

2. Nahtankersystem nach Anspruch 1, wobei die Nahterfassungskonfiguration eine Schlaufe (28) umfasst, durch die das Nahtmaterial (42) geführt werden kann.

3. Nahtankersystem nach Anspruch 1, wobei die eine oder die mehreren Halteverstärkungen (32) Schraubengewinde (41) umfassen.

## Revendications

1. Système d'ancrage de sutures, comprenant :
un corps d'ancrage distal (12) ayant des filets (22) sur celui-ci pour permettre de visser le corps d'ancrage distal (12) dans un os, et une fixation de sutures (18) ;
un élément d'enfoncement (52) pour enfoncer le corps d'ancrage distal (12) dans l'os, présentant un corps allongé de dispositif d'enfoncement, le corps d'ancrage distal (12) étant attaché de manière amovible à une extrémité distale (50) de celui-ci ; et
un corps d'ancrage proximal (30) ayant un alésage central (34) passant à travers celui-ci et un ou plusieurs éléments de renfort (32) s'étendant depuis le corps d'ancrage proximal (30) ;
**caractérisé en ce que** :
le corps d'ancrage distal (12) présente également un fil-guide (20) s'étendant en direction proximale depuis le corps d'ancrage distal (12) et attaché de manière amovible à celui-ci, le fil-guide (20) s'étendant le long du corps de dispositif d'enfoncement ;
le système comporte un élément de passage de sutures (24) comprenant un organe flexible allongé (26) vissé à travers la fixation de sutures (18) et présentant une configuration de capture de sutures distale (28) ;
l'alésage central (34) du corps d'ancrage proximal (30) est dimensionné pour recevoir le fil-guide (20) de telle sorte que le corps d'ancrage proximal (30) puisse être enfoncé dans l'os une fois que le dispositif d'enfoncement (52) a été enlevé ; et
le ou les éléments de renfort (32) s'étendant depuis le corps d'ancrage proximal (30) sont prévus pour piéger une suture (42) qui a été tirée à travers la fixation de sutures (18) par l'élément de passage de sutures (24) entre le corps d'ancrage proximal (30) et l'os.

2. Système d'ancrage de sutures selon la revendication 1, dans lequel la configuration de capture de sutures comprend une boucle (28) à travers laquelle peut être passée la suture (42).

3. Système d'ancrage de sutures selon la revendication 1, dans lequel le ou les éléments de renfort (32) comprennent des filets de vis (41).
